# EUROPEAN PATENT SPECIFICATION

(11) **EP 0 364 606 B1**
(45) Date of publication and mention of the grant of the patent: **16.03.1994**
(21) Application number: 89904606.4
(22) Date of filing: 13.04.1989
(51) Int. Cl.: C12N 5/00, C12M 3/00

(54) **PROCESS FOR CULTURING ANIMAL CELLS ON A LARGE SCALE AND PROCESS FOR PREPARING SUPPORTING SUBSTRATE FOR THAT PROCESS**
VERFAHREN ZUR ZÜCHTUNG TIERISCHER ZELLEN IN GROSSEM MASSSTAB UND VERFAHREN ZUR HERSTELLUNG VON TRÄGERMATERIAL DAFÜR
PROCEDE POUR LA MISE EN CULTURE DE CELLULES ANIMALES A GRANDE ECHELLE, ET PROCEDE POUR LA PREPARATION DU SUBSTRAT DE SUPPORT A CET EFFET

(30) Priority: 18.04.1988 JP 96528/88
(43) Date of publication of application: 25.04.1990
(73) Proprietor: NITTA GELATIN INC., Osaka-shi Osaka 541 (JP)
(72) Inventor: ENAMI, Junpei, Shimotsuga-gun Tochigi 321-02 (JP); KONDO, Naohito, Osaka 581 (JP); TAKANO, Toshikazu, Ikoma-gun Nara 636 (JP); SUZUKI, Kaneo, Kashihara-shi Nara 634 (JP)
(74) Representative: Pacitti, Pierpaolo A.M.E.
(86) International application number: PCT/JP89/00404
(87) International publication number: WO 89/10397

(56) References cited:
- EP-A- 0 173 915
- WO-A-87/00197
- JP-A- 633 786
- JP-A- 6 225 974
- JP-A-55 157 502
- JP-A-62 175 172

## Description

### Technical Field

The present invention relates to methods for cultivation of animal cells on a large scale and for making supporting substrata for the cultivation.

### Background Art

Animal cells are classified into two categories; one is anchorage-dependent cells which grow or proliferate only after attachment to supporting substrata, and the other is anchorage-independent cells which do not require attachment to supporting substrata and can grow even in a suspension state. The method for cultivation of the anchorage-dependent cells on a large scale differs from that for the anchorage-independent cells in requirment of a suitable supporting substrate for cell attachment, and various kinds of two- and three-dimensional culture methods, depending on the difference in the supporting substrate structure or the substrate material used, have been proposed [ Japanese national publication of translation of international patent application, showa 62-502936 ( International publication number WO 86/05811 ), Cytodex ( commertial name for a product from Pharmacia Fine Chemicals Co., Ltd. ), and Japanese official patent provisional publication, showa 63-209581 ] .

In the two-dimensional culture methods, animal cells attach to the surface of the supporting substrate and grow on the surface to form a monolayer of cells. For the two-dimensional culture methods, a number of methods have been proposed wherein the purposes are as follows: increase in cell density of cells cultured, enhancement of product secretion in cultured cells, simplifying of recovery and purification of the cultivated products, and the like.

On the other hand, some of the present inventors have proposed a large-scale cultivation method wherein anchorage-dependent animal cells are cultured three-dimensionally by using collagen gels as supporting substrata ( Japanese official patent provisional publication, showa 62-175172; Japanese official patent provisional publication, showa 62-171680; International publication number WO 87/04458; USSN 110749; and the European patent application, publication number 0258441 ). In this method, the cells are embedded in collagen gels and grow three-dimensionally in the gels. Since collagen is possible to make in vitro surroundings which is very close to in vivo surroundings, it has been proved that the collagen gel culture method is possible to culture some kinds of cells, which are unable to culture by monolayer culture methods and by three-dimensional culture methods using other supporting substrata than collagen. Therefore, the three-dimensional culture method using collagen gel has, compared to two-dimensional culture methods and methods using other three-dimensional substrata, such advantages that are not only increase in producibility arising from increase in cell attachment area, which is due to the fact that is three-dimension, and simplicity of culture due to cell fixation, but also increase in the kind of cells possible to culture.

Considering these advantages, the present inventors studied development of a method for large-scale cultivation in which animal cells are embedded in collagen gels and cultured three-dimensionally. As a result, it was found that this culture method has at least one problem among the following ① ∼ ③.
① If collagen gel is soft, it is hard to handle and apt to be hurted.
② As the collagen gel shrinks with cell growth, a long-term cultivation is impossible.
③ When a collagen gel of high gel strength is used as a supporting substrate, said ① and ② problems may be solved. However, if gel strength of the collagen gel is high, there appear new problems such as decrease in cultivating rate of cells or limitation of the kind of animal cells possible to be cultivated.

These problems affect badly not only for convenience in treatment, but for the cell growth, so that should be of necessity solved.

Accordingly, the first subject of the present invention is to provide a method for large-scale cultivation of animal cells wherein collagen gels in which animal cells are embedded are easily handled, shrinkage of said collagen gels being accompanied with cell growth can be prevented, and collagen gels having a suitable gel strength as supporting substrata can be used. Futhermore, the present invention has a second subject which is to provide a method to produce easily supporting substrata for using in said method for large-scale cultivation.

### Disclosure of Invention

A method for large-scale cultivation of animal cells relating to claim 1 in the present invention is, in order to solve said first subject, characterized by that a collagen gel in which animal cells are embedded is covered by a protective coating and the gel is supported with the protective coating. With this, the protective coating protects the collagen gel inside the coating and thereby its gel shape is kept. For this reason, the collagen gel with a gel strength suitable for a cell to be cultivated can be used.
Further, since the collagen gel in which animal cells are embedded is supported with said protective coating from the outside, the collagen gel does not shrink with the cell proliferation ( which have already produced a great number of cells ), so that cell proliferation is maintained for a long period.

A method for producing a supporting substrate for the cultivation relating to claim 2 in the present invention is, in order to solve said second subject, characterized by that a collagen solution in which animal cells are suspended and a coating-forming agent solution forming a protective coating are fed at the same time from the inner and the outer tubes of a double layer nozzle, respectively, and then, after the collagen solution is covered with the coating-forming agent solution, the coating-forming agent solution is hardened by putting in a hardening solution to make a protective coating and to undergo gelation, and thereby said animal cells are embedded in a collagen gel being covered with the protective coating. That is, by vomiting from the double layer nozzle, a collagen solution suspended with animal cells comes in contact with the coating- forming agent solution in a solution state and so, is covered by this agent solution, and under this condition the agent solution is hardened in the hardening solution to make a protective coating.

The collagen is a favorable agent for formation of a supporting substrate in points that, since it is a fiber protein existing everywhere in an animal body, it realizes the in vivo surroundings in vitro ( in an outside of the living body ) by being transformed into a gel and being used as a supporting substrate for a cell. The kind of collagen being used for this invention is not especially limited and various types of collagens can be used.

Collagen is used as a solution. The collagen solution is prepared as to have physiological salt concentration and pH and the cell is suspended in the solution. To prepare a collagen gel using this collagen solution and to embed the cell in the gel, the collagen solution is transformed into a gel, for example, with warming at 25 ∼ 37 °C and the cell suspended is embedded in the collagen gel. The collagen solution can be treated by adding, if necessary, various components ( for example, a physiologically active substance such as a serum, a cell-growing factor, and a hormone etc. ).

That an animal cell is embedded in a collagen gel is to support the cell undergoing the anchorage-dependent proliferation and to bring the cell close to the in vivo surroundings in order to that the cell can be proliferated in a three dimensional way. By doing in this way, the cell can be proliferated in a three dimensional way, it can be cultivated in high density, in large quantity and also, for a long period as compared with the previous method. As well, cell differentiation can be derived.

The collagen gel, as far as it has a shape suitable for large-scale cultivation, is not especially limited in shape. For example, are cited a particle shape such as a sphere, a cylinder, an ellipsoid, a cube, a rectangular parallelepiped shape, and an indefinite shape etc., a noodle shape, a sheet shape and so forth. If it is a particle shape, since a surface area per volume can be enlarged, it is profitable for material exchange between the inside and outside. The shape size is not especially limited and, for example, preferred is a range of 0.1 ∼ 10 mm in the length and side directions, respectively. If the size is larger than this range, there is a possibility that either transmission of a nutrition component or a superannuated product or gas exchange through the protective coating between collagen gel and the outside cultivating solution is delayed or does not take place at all, and the cell proliferation may be disturbed. If smaller than the range, there may be afraid inconvenience that, when the cultivating solution is exchanged, a solid is floating and dispersing and, at filtering, blocking takes place. Besides, a collagen gel attaching to a protective coating is preferred to have a beads shape of 3 mm or less in diameter or a related shape. This is because, when the cultivating solution is exchanged, the collagen gel is sufficient in size for easy separation from a cultivating solution where the collagen gel is soaked, and the material exchange between an inside and an outside of the collagen gel becomes facile. According to the method for large-scale cultivation of animal cells in the present invention, it is possible to cultivate large quantity in a small space and also, to take out with high efficiency a cell or a product.

The protective coating is favorable if it has, to protect and support a collagen gel, such special properties as hardness, capability of hardening during a short period, absence of toxity against cells, and facile transmission of a nutrition component into the cultivating solution and a metabolite ( or a product ) of cells. More favorable is that, in addition to those, if it can be dissolved again under a condition that the cell is not hurted, the cell can be recovered as alive after treating with large-scale cultivation.

Although the protective coating is not especially defined, for example, a coating is used which is prepared in a thin-layer state by hardening of a high molecular weight compound such as alginic acid, alginic acid salt, carrageenan, locustbeen gum, chitin, a chitin derivative, agarose, polyurethane, polylysine, and polyvinyl alcohol ( in this invention, a high molecular weight compound of these kinds is called as 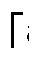 a coating-forming agent 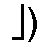 ). The coating-forming agent is not especially defined, if it is capable of hardening under a condition that a cell is not hurted and also, is harder than a collagen gel. Besides, from a point of that a jointing force between the collagen gel and the protective coating is further increased, preferred is the one having superior affinity with collagen as a coating-forming agent. Sickness of the protective coating is not especially defined, preferred is to be in a range of 0.01 ∼ 10 mm and more preferred is to be in a range of 0.1 ∼ 1.0 mm. If the protective coating is thicker than the upper limit of the range, the material exchange may be disturbed. If thinner than the lower limit, sufficient formation of a protective coating becomes difficult and, even if the coating is formed, there may be a case that the coating is hurted or the collagen shrinkage is not stopped.

For the protective coating, is preferred to cover 50 % or more of a total surface of the collagen gel and more preferred 100 % covering. If the covering is less than 50 %, physical strength is weak and handling becomes hard.

When a collagen gel attached to a protective coating and formed in a noodle or a sheet shape is cut after the forming, the covering percentage becomes less than 100 %. Also, the covering percentage is less than 50 %, the collagen gel shrinkage sometimes can not be stopped.

The protective coating is obtained, for example, by that the outside surface of a collagen gel in which animal cells are embedded or of a drop of a collagen solution suspended with animal cells is wrapped in a coating-forming agent solution using for formation of a protective coating and then, said coating-forming agent solution is hardened. When the outside surface of a drop of a collagen solution is wrapped in a coating-forming agent solution, the collagen solution undergoes gelation after a protective coating being formed or together with the coating formation. The protective coating covers the collagen gel, firmly joints with it, and supports it from the outside. By this, collagen shrinkage is prevented. Besides, since the collagen gel is covered with the protective coating, even if it collides with other things or comes in contact with, the animal cell as well as the collagen gel are protected.

The coating-forming agent solution may contain either only one coating-forming agent, or together other kinds of coating-forming agents, a buffer solution, a culture solution, a serum, or other additives.

Hardening of the coating-forming agent solution can be carried out in any way, for example, by putting in a hardening solution. This hardening solution is properly prepared according to the kind of a coating-forming agent solution. At this time, depending upon a hardening condition for the coating-forming agent solution, the hardening solution is used by suitably selecting from, for example, a salt solution, a crosslinking agent solution, or water ( for example, cold water etc. ). A preferred hardening solution is the one not giving any hurt for a cell. If more concrete examples are cited from combination of the coating-forming agent solution with the hardening solution and, if the coating-forming agent solution is a sodium alginate solution, a solution containing a two-valents metallic ion or a solution made by a polycation such as chitin and chitosan is used. Especially, a calcium ion is used as a metallic ion. If the coating-forming agent solution is a solution of sodium alginate, and for example, in a case of continuous gel formation where the below-described double layer nozzle is used, gelation rapidly proceeds with a calcium ion of 50∼ 70 mM ( millimoles ). In a case of that a chitosan solution is used as a hardening solution for an aqueous solution of sodium alginate, a thin membrane is rapidly formed on a dropping particle surface with a reaction of chitosan with alginic acid and a membrane having strength sufficient for operation can be formed within a short time, and there is an effect capable of further supporting an alginic acid gel from the outside.

Besides, in a case of that alginic acid, alginic acid salt, and salts of alginic acid derivatives etc. are used as a coating-forming agent, a compound undergoing rapidly gelation with a calcium ion of 0.1 M or less is favorably used. This is because calcium ion concentration of this order is in a range similar to that of a living body ( the in vivo surroundings ) or a range of that a cell is not hurted.

In the present invention, the hardening comprises not only gelation of the above-described natural high molecular weight substance, but also formation of a crosslinking structure of the synthetic high molecular weight substance.

Since the protective coating and the collagen gel both are able to contain a large amount of water, material exchange between these inside and outside ( for example, breathing, intake of nutrition, extretion, and release of secretion etc. ) is possible and suitable for cultivating a cell. For example, if the protective coating is a product obtained from hardening of said natural high molecular weight substance, said product is a gel and able to hold a large amount of water. If the protective coating is a product obtained from hardening of a polyurethane, since a number of fine continuous pores are formed by carbon dioxide gas generating during hardening, a large amount of water can be held in the pores. Also, if the protective coating is a polylysine or a polyvinyl alcohol, a large amount of water can be held in a crosslinking structure of these products obtained from hardening.

Besides, to carry out a method for large-scale cultivation of animal cells relating to the invention in claim 1, the animal cells being embedded in a collagen gel, which is covered with a protective coating , are not especially defined, but for example, it can be obtained from a method for producing a supporting substrate for cultivation in the invention in claim 2. That is, by using a double layer nozzle composed of an inner tube and an outer tube, a collagen solution suspended with animal cells is supplied continuously or intermittently for the inner tube through a pipe etc. and, at the same time, a coating-forming agent solution is supplied continuously for the outer tube through a pipe etc. and then, from the double layer nozzle both the solution is dropped or vomited with pushing. Besides, the double layer nozzle is recommended to be installed in a tightly closed vessel. For this double layer nozzle may be used the one in which a front top of the inner tube is stretched longer than a front top of the outer tube, the one in which both the tops are in the same length, or the one in which a front top of the inner tube is shorter than a front top of the outer tube. The collagen solution getting out from the inner tube is, in part or wholly, wrapped by a coating-forming agent solution getting out from the outer tube and, it takes a sphere or a noodle shape. Under these conditions, when the wrapped collagen solution of such a shape is put into said hardening solution, the coating-forming agent solution in the outer layer undergoes hardening to form a protective coating. Then, a thus-formed product is separated from the hardening solution and is washed by a physiological salt solution ( for example, a Hanks' solution ) to remove a metallic ion such as an excess calcium ion etc. Then, the cultivation is performed with soaking in a cultivating solution and with stirring or circulating of the cultivating solution. Furthermore, the gelation of the collagen solution, if it is before cultivation, can be carried out at any time.

Cultivation is performed by that a collagen gel in which animal cells are embedded are covered with a protective coating and are soaked in the cultivating solution. Component and concentration of the cultivating solution and cultivating temperature are set by properly selecting from those suitable for the embedded animal cells.

The means for large-scale cultivation are not especially limited and for those are cited, for example, proper stirring and exchanging of a culture solution with placing in a bottle of a stirrer type, a bottle of a roller type, a bottle of a propeller type, or a bottle of an air agitation type, and proper filling and holding in a column capable of circulating a culture solution and then, circulating the solution in the column. Even if large-scale cultivation of this kind is used, the collagen gel is not hurted and the treatment is easy. Because of this, a large volume of vessel can be used as a vessel to cultivate an animal cell and with good efficiency.

Furthermore, by mixing a heavy substance with a protective coating and/or a collagen gel, specific gravity can be adjusted and also, separation of cells from a cell-cultivating solution, when a culture solution is exchanged, can be done easily.

The method for large-scale cultivation of animal cells relating to the claim 1 invention is such as mentioned above, so that all the below-described effects ( a )∼ ( c )are held.
( a ) Since a collagen gel is protected by a protective coating, handling such as transferring it to a cultivation apparatus is easy, and the gel is not easily hurted when a culture solution is exchanged or circulated.
( b ) Since shrinkage of a collagen gel being accompanied with cell growth is prevented, the cell is kept for a long time.
( c ) Since said ( a ) and ( b ) effects are obtained independent of the magnitude in gel strength of a collagen gel, a gel strength suitable for an animal cell to be cultivated can be chosen and thereby, the kind of animal cells possible to cultivate becomes wide.

A method for producing a supporting substrate for cultivation relating to the claim 2 invention can easily afford an animal cell embedded in a collagen gel which is attached to a protective coating.

### Brief Description of the Drawings

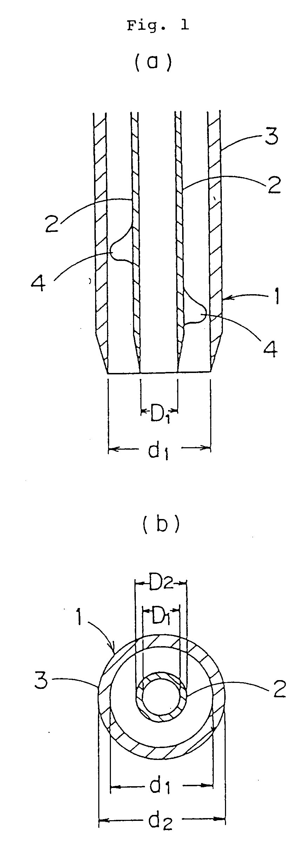

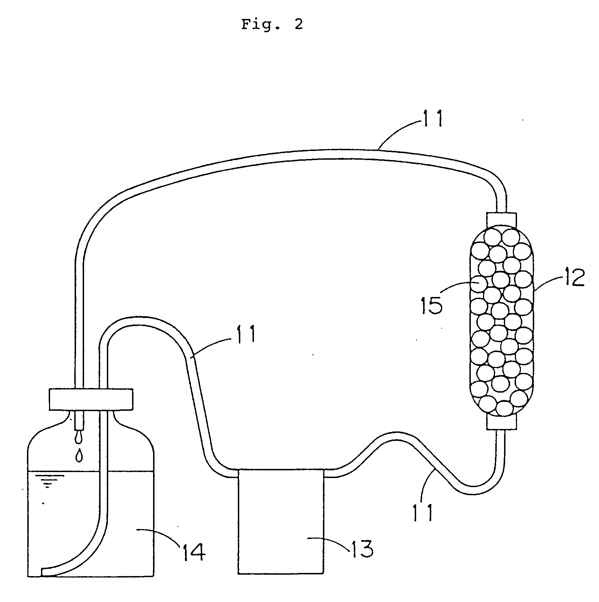

Fig. 1 shows a double layer nozzle used for examples; and Fig. ( a ) is a longitudinal sectional view and Fig. ( b ) is a transverse sectional view. Fig. 2 is a model view for an apparatus for large-scale cultivation used in examples.

### Best Mode for Carrying Out the Invention

Hereinafter, concrete examples and reference examples in the present invention are shown, but the invention is not limited within the below-described examples.

### Example 1

Cellmatrix I-A ( acid-soluble collagen; concentration 3.0 mg/ml; pH 3.0; Nitta Gelatin Inc. ) 8 parts by volume was mixed with Ham's F 12 culture medium ( 10 times concentration ) 1 part by volume, a reconstitution buffer solution ( prepared by dissolving 2.2 g of sodium hydrogencarbonate and 4.77 g of HEPES in 100 ml of a 50 mM aqueous sodium hydroxide solution ) 1 part by volume, and fetal calf serum 1 part by volume, and to the obtained mixed collagen solution was added a mouse mammary tumore cell line suspension 1 part by volume. The resulting solution was kept at 4 °C to give a collagen solution containing animal cells which was used as an inner solution.

On the other hand, an 1 % aqueous solution of sodium alginate was used as an outer solution. By using a double orifice nozzle 1 having the same center as shown in Fig. 1, the outer solution was continuously fed from the outer tube 3, while the inner solution was intermittently fed from the inner tube 2, and the resulting drops were dropped into a 1 % aqueous calcium chloride dihydrate solution to form beads. After standing for 10 ∼ 20 minutes, the beads were recovered by filtration using a nylon mesh and washed in Hanks' solution without calcium and magnesium to remove excess calcium chloride.

In this procedure, the dropping rate was 50 drops per minute and the total volume of the outer and inner solution 30 µl per drop and the volume ratio between the inner solution and outer solution 1 to 2. As shown in Fig. 1 ( a ) and ( b ), for the nozzle 1 the inner tube 2 and the outer tube 3 are arranged in a mode having the same center hold. The internal diameter ( D₁ ) and external diameter ( D₂ ) of the inner tube 2 are 0.30 and 0.50 mm, respectively, and the corresponding diameters ( d₁ ) and ( d₂ ) of the outer tube 3 are 0.85 and 1.20 mm, respectively. In Fig. 1, 4 shows a supportinng material for the tube center.

Next, the beads were warmed in Dulbecco's modified Eagle's medium for 60 minutes at 37°C to undergo gelation of the inner collagen, in which cells were dispersed, to obtain alginate-coated collagen gel beads, whose average diameter was approximately 3.5 mm.

The obtained collagen gel beads which contain cells and were covered with alginic acid were subjected to shaking culture using Dulbecco's modified Eagle's medium containing 10 % fetal calf serum and 10 mM HEPES in a flask maintained at 37°C

### Reference example 1

When the double-layer gel beads obtained from example 1 is soaked in Hanks' solution without calcium and magnesium and with 10 mM EDTA for 7∼ 15 minutes, the outer layer alginate was solved to give beads of cell-containing collagen gel alone, which did not contained a protective coating. With this collagen beads, culture was carried out in the same manner as in example 1.

The collagen beads without a protective coating( reference example 1 ) were shrinked from about 2.6 mm to about 1.1 mm in average diameter after cutivation for 12 days. In contrast, the alginate-coated collagen gel beads ( example 1 ) showed no change in the average diameter even on the 12th day after cultivation was initiated.

Table 1 presents the results obtained from measurements of cell number in both the beads. Cell proliferation is maintained in the alginate-coated collagen gel beads, whereas it is recognized that the proliferation reachs the uppermost limit on the 7th day after cultivation has been initiated in the case of beads without a protective coating. Additionally, a dendritic outgrowth was observed for mouse mammary tumor cell line in the alginate-coated collagen gel beads. This shows that the collagen gel is a very suitable supporting substrate for the cultivation of anchorage-dependent cells.

**Table 1**

| Cultivating days | DNA ( µg/bead ) | |
|---|---|---|
| | Reference example 1 | Example 1 |
| 0 | 0.07 | 0.07 |
| 5 | 0.20 | 0.33 |
| 7 | 0.69 | 1.01 |
| 10 | 0.60 | 1.12 |
| 12 | 0.68 | 1.26 |

### Example 2

Except that, in example 1, a mouse fibroblast was used instead of a mouse mammary tumor cell line, the same treatment as in example 1 gave alginate-coated collagen gel beads containing cells. As seen in Fig. 2, 1000 pieces of beads 15 ( volume was about 40 cm³ ) were packed in a polystyrene column 12. As seen in Fig. 2, the column 12 was connected with tube 11 and the culture medium 14 was circulated by the pump 13. The apparatus shown in Fig. 2 was mounted in a CO₂ incubater maintained at 37°C and the culture medium 14 used in example 1 was continously circulated for cultivation. As a result, without beads destruction, a cell was able to be cultivated for about 1 month and thus, a strong physical character of the beads was confirmed. By the way, the culture medium exchange was carried out in a manner that an amount of the circulating culture medium was about 1 ml per minute, an amount of the using culture medium was 50 ml per day at the beginning and 500 ml per day at the last, and a bottle for the medium was exchanged every day.

That the cell can be cultivated for a long period in the cultivation method using a column as shown in example 2 shows such effects as the below-described ( A )∼ ( C ) and is very useful as a system for large-scale cultivation of animal cells.
( A ) Culture medium exchange can be carried out very easily.
( B ) Recovery of products from cells can be carried out easily and effectively.
( C ) Large-scale cultivation can be carried out in a compact space.

### Example 3

Except that, in example 1, the cell was replaced by a mouse fibroblast and the inner solution and the outer solution were continuously, at the same time, pushed out, a procedure same as that for example 1 gave a product of a double layer noodle shape, which was washed and then, warmed at 37 °C for 1 hour to cause gelation of the inner collagen solution. This gel was a double layer gel of a noodle shape having a diameter of about 1 mm.

The product obtained was subjected to static cultivation in a CO₂ incubator at 37 °C by using the culture medium used for example 1.

On the second day of cultivation, developing fibroblast was recognized.

### Example 4

Except, in example 1, a 0.5 % aqueous chitosan solution was used, instead of a 1.0 % aqueous calcium chloride dihydrate solution, as a hardening solution for a 1.0 % aqueous sodium alginate solution, that was the outer solution, a procedure same as that for example 1 gave alginate-coated collagen beads containing cells.

When the obtained alginate-coated collagen beads containing cells was cultivated similarly to example 1, it was confirmed that the beads has a sufficient strength.

### Industrial Applicability

The method for large-scale cultivation of animal cells in the present invention, as far as it is an animal cell, can be applied for both the anchorage-dependent cell and the anchorage-independent cell. In particulars the effect is significant if the method is applied for cultivation of the anchorage-dependent cell. Even in the case of anchorage-dependent cells, if the cell growth is not disturbed with the collagen shrinkage or, if large shrinkage does not take place with progressing proliferation, a coating-forming agent can be selected without attention to joining the collagen gel with the protective coating. For example, an agent like agarose, which has a weaker joining force compared with that of alginic acid etc., can be used as a coating-forming agent.

According to a method for large-scale cultivation of animal cells, animal cells cultivated in large quantity can be obtained within a short time and in a small space. Also, if a cell is cultivated in a collagen gell according to this method, since appearance of an animal cell function being cultivated is very active, various kinds of cell-produced materials can be obtained with high efficiency. As produced materials of such kinds, for example, are cited vaccine, enzyme, hormone, antibody, nucleic acid, and so forth. Also, the cell itself can be used, by being greatly proliferated, for several kinds of purposes.

The method to take out a cell after cultivation, for examplle, if the protective coating is a calcium alginate coating, the calcium ion is removed with a chelate agent such as EDTA and EGTA etc. and thus, the alginate coating can be easily dissolved. The inner collagen gel can be decomposed by using collagenase etc., so that the cell can be recovered as alive.

Also, for recovery of a material produced from the cell, which is cultivated in large quantity, for example, is sufficient to collect the material together with a culture solution, which comes into the culture solution passing through the protective coating.

Besides, when a method for large-scale cultivation of animal cells in the present invention is carried out, the animal cell, which is embedded in collagen gel covered by a protective coating, is not especially limited but, for example, can be obtained by a method for making supporting substrata for the cultivation in the present invention. According to this method, an animal cell of that kind can be arranged with high efficiency and only an aqueous solution can be used without using an organic solvent. In addition, contamination from outside is easily prevented and a sterile condition is easily maintained.

## Claims

1. A method for cultivating animal cells embedded in a collagen gel, substantially covered with a protective coating which is formed by a coating forming agent solution comprising at least one of alginic acid, alginic acid salt and alginic acid derivative salt, and is hardened by contact with a hardening agent solution comprising a calcium salt, wherein the collagen gel is formed by gelation of a collagen solution having the cells suspended therein, characterised in that the collagen solution is emitted from an inner tube of a double orifice nozzle, the coating forming agent solution is simultaneously emitted from an outer tube of the double orifice nozzle thereby substantially covering the collagen solution, the coating forming agent solution covering the collagen solution is hardened by contact with the hardening agent solution to form the protective coating, and the collagen solution substantially covered with the protective coating is gelled.

2. A method for producing a supporting substrate for use in the cultivation of large quantities of animal cells embedded in a collagen gel comprising:
emitting from an inner tube of a double orifice nozzle a suspension of animal cells in a collagen solution;
simultaneously emitting from an outer tube of said double orifice nozzle a coating forming agent solution covering the collagen solution with the coating forming agent solution, the coating forming agent solution comprising at least one of alginic acid, alginic acid salt and alginic acid derivative salt,
hardening the coating forming agent solution by contact with a hardening solution comprising a calcium salt thereby forming a protective coating; and,
allowing said collagen solution to undergo gelation.

## Patentansprüche

1. Verfahren zur Züchtung tierischer Zellen, die in ein Kollagengel eingebettet sind, das mit einer Schutzbeschichtung im wesentlichen bedockt ist, die durch eine Lösung eines eine Beschichtung ausbildenden Mittels ausgebildet ist, welches wenigstens eines von Alginsäure, Alginsäuresalz und Alginsäure-Derivatsalz enthält, und die durch Herührung mit einer Härtemittellösung gehärtet wird, welche ein Kalziumsalz enthält, wobei das Kollagengel durch eine Gelierung einer Kollagenlösung mit darin suspendierten Zellen augebildet ist, dadurch gekennzeichnet, daß die Kollagenlösung von einem Innenrohr einer Düse mit zwei Mündungen ausgeströmt wird, die das Mittel zur Ausbildung der Beschichtung enthaltende Lösung gleichzeitig von einem Außenrohr der Düse mit den beiden Mündungen ausgeströmt und dadurch die Kollagenlösung im wesentlichen abgedeckt wird, die das Mittel zur Ausbildung der Beschichtung enthaltende Lösung, welche die Kollagenlösung abdeckt, durch Berührung mit der Härtemittellösung ausgehärtet wird, um die Schutzbeschichtung auszubilden, und die Kollagenlösung, die mit der Schutzbeschichtung im wesentlichen abgedeckt ist, geliert wird.

2. Verfahren zur Herstellung eines Support-Substrats zur Verwendung bei der Züchtung von großen Mengen von tierischen Zellen, die in ein Kollagengel eingebettet sind, bestehend aus:
Ausströmen einer Suspension von tierischen Zellen in einer Kollagenlösung von einem Innenrohr einer Düse mit zwei Mündungen;
gleichzeitiges Ausströmen einer ein Mittel zur Ausbildung einer Beschichtung enthaltenden Lösung von einem Außenrohr der Düse mit den beiden Mündungen für eine Abdecken der Kollagenlösung mit dar Lösung des eine Beschichtung ausbildenden Mittels, wobei die Lösung des eine Beschichtung ausbildenden Mittels wenigstens eines von Alginsäure, Alginsäuresalz und Alginsäure-Derivatsalz enthält;
Aushärten der ein Mittel zur Ausbildung einer Beschichtung enthaltenden Lösung durch eine Berührung mit einer Aushärtelösung, die ein Kalziumsalz enthält, sodaß dadurch eine Schutzbeschichtung ausgebildet wird; und
Ermöglichung einer Gelierung der Kollagenlösung.

## Revendications

1. Procédé pour la mise en culture de cellules animales enrobées dans un gel de collagène, essentiellement recouvert par un revêtement protecteur formé par une solution d'un agent de formation dc revêtement comprenant au moins, soit un acide alginique, soit un sel d'acide alginique, soit un sel dérivé de l'acide alginique, et qui est durci par contact avec une solution d'un agent de durcissement comprenant un sel de calcium, dans lequel le gel de collagène est formé par gélification d'une solution de collagène dans laquelle les cellules sont en suspension, caractérisé en ce que la solution de collagène est émise à partir du tube intérieur d'une buse à double orifice, la solution d'un agent de formation de revêtement étant simultanément émise à partir du tube extérieur de la buse à double orifice, recouvrant ainsi substantiellement la solution de collagène, la solution d'un agent de formation de revêtement recouvrant la solution de collagène étant durcie par contact avec la solution d'un agent de durcissement pour former le revêtement protecteur, la solution de collagène substantiellement recouverte par le revêtement protecteur étant gélifiée.

2. Procédé pour la fabrication d'un substrat de support utilisable pour la mise en culture de cellules animales à grande échelle enrobées dans un gel de collagène, comprenant :
l'émission à partir du tube intérieur d'une buse à double orifice d'une suspension de cellules animales dans une solution de collagène,
simultanément, l'émission à partir du tube extérieur de ladite buse à double orifice d'une solution d'un agent de formation de revêtement pour recouvrir la solution de collagène avec la solution d'un agent de formation de revêtement, la solution d'un agent de formation de revêtement comprenant su moins un acide alginique, ou un sel d'acide alginique, ou un sel dérivé de l'acide alginique,
le durcissement de la solution d'un agent de formation de revêtement par contact avec une solution de durcissement comprenant un sel de calcium, en formant ainsi un revêtement protecteur, et
en laissant la solution de collagène effectuer une gélification.
